# FASCICULE DE BREVET EUROPEEN

(11) **EP 0 432 254 B1**
(45) Date de publication et mention de la délivrance du brevet: **23.04.2003**
(21) Numéro de dépôt: 90910749.2
(22) Date de dépôt: 05.07.1990
(51) Int. Cl.: C12N 15/57, C12N 9/64, G01N 33/573, C12Q 1/68

(54) **ACIDE NUCLEIQUE CODANT POUR L'ENZYME DE CONVERSION DE L'ANGIOTENSINE (ECA) TESTICULAIRE HUMAINE, ET SES APPLICATIONS, NOTAMMENT POUR LE DEPISTAGE IN VITRO DE CETTE ENZYME DANS L'ORGANISME**
NUKLEINSÄURE, DIE FÜR DAS MENSCHLICHE TESTIKULARE ANGIOTENSIN KONVERTIERENDE ENZYM (ACE) KODIERT, UND SEINE ANWENDUNGEN, INSBESONDERE FÜR DIE IN VITRO-DETEKTION DES ENZYMS IM ORGANISMUS
NUCLEIC ACID CODING FOR THE HUMAN TESTICULAR ANGIOTENSIN CONVERSION ENZYME (ECA) AND ITS APPLICATIONS, IN PARTICULAR FOR THE IN VITRO DETECTION OF SAID ENZYME IN THE ORGANISM

(30) Priorité: 05.07.1989 FR 8909062
(43) Date de publication de la demande: 19.06.1991
(73) Titulaire: INSTITUT NATIONAL DE LA SANTE ET DE LA RECHERCHE MEDICALE (INSERM), 75654 Paris Cédex 13 (FR)
(72) Inventeur: SOUBRIER, Florent, F-75016 Paris (FR); ALHENC-GELAS, François, F-75015 Paris (FR); HUBERT, Christine, F-92310 Sèvres (FR); CORVOL, Pierre, F-75007 Paris (FR)
(74) Mandataire: Gutmann, Ernest
(86) Numéro de dépôt international: FR9000513
(87) Numéro de publication internationale: WO91000354

(56) Documents cités:
- PROCEEDINGS OF THE NATIONAL ACADEMY OF SCIENCES OF USA. vol. 85, no. 24, décembre 1988, WASHINGTON US pages 9386 - 9390; SOUBRIER, F. et al.: "Two putative active centers in human angiotensin I-converting enzyme revealed by molecular cloning." voir le document en entier SA 38761 030(cité dans la demande)
- BIOCHEMICAL AND BIOPHYSICAL RESEARCH COMMUNICATIONS. vol. 128, no. 1, 16 avril 1985, DULUTH, MINNESOTA US pages 457 - 463; LANZILLO, J.J. et al.: "Human testicular angiotensin-converting enzyme is a mixture of two molecular weight forms. Only one is similar to the seminal plasma enzyme" voir le document en entier SA 38761 030
- FEBS LETTERS. vol. 252, no. 1,2, juillet 1989, AMSTERDAM NL pages 99 - 104; LATTION, A-L. et al.: "The testicular transcript of the angiotensin I-converting enzyme encodes for the ancestral, non-duplicated form of the enzyme." voir le document en entier
- PROCEEDINGS OF THE NATIONAL ACADEMY OF SCIENCES OF USA. vol. 86, no. 20, octobre 1989, WASHINGTON US pages 7741 - 7745; EHLERS, M.R.W. et al.: "Molecular cloning of human testicular angiotensin-converting enzyme: the testis isozyme is identical to the C-terminal half of endothelial angiotensin-converting enzyme" voir le document en entier

## Description

L'invention concerne un acide nucléique codant pour l'enzyme de conversion de l'angiotensine (ECA) dans les cellules germinales de testicules humains ainsi que des vecteurs contenant cet acide nucléique et l'utilisation de ces derniers pour la production de cette enzyme. L'invention concerne également les applications de cet acide nucléique, notamment pour le dépistage in vitro de cette enzyme dans l'organisme.

L'ECA, ou peptidyl dipeptidase A (EC 3.4.15.1), ou encore kininase II, joue un rôle important dans la régulation de la pression artérielle, en hydrolysant l'angiotensine I (peptide inactif libéré après clivage de l'angiotensinogène par la rénine) en angiotensine II vasopressive jouant un rôle majeur dans la régulation de la pression artérielle (SKEGGS, L.T., et al (1956) J. Exp. Med., 103, 295-299).

L'inhibition de l'activité de l'ECA par l'EDTA et les chélateurs de métaux, indique qu'il s'agit d'une métallopeptidase, plus particulièrement d'une peptidase à zinc, capable d'hydrolyser, non seulement, l'angiotensine I, mais aussi la bradykinine (un peptide vasodilateur et natriurétique qu'elle transforme en un heptapeptide inactif), et de nombreux autres peptides à activité biologique (YANG, H.Y.T. et al (1970) Biochim. Biophys. Acta, 214, 374-376 ; ERDOS, E.G., et al (1987) Lab. Invest., 56, 345-348).

L'ECA est une peptidase largement distribuée dans l'organisme, que l'on retrouve par exemple sous forme d'enzyme membranaire à la surface des cellules endothéliales vasculaires, et des cellules épithéliales rénales, ainsi que sous forme d'enzyme circulant dans le plasma (ERDOS, et al (1987) sus-mentionné ; CARDWELL, P.R.B. et al, (1976) Science, 191, 1050-1051 ; RYAN, U.S. et al (1976) Tissue Cell, 8, 125-145).

Des méthodes de purification de l'ECA endotheliale humaine ou animale ont déjà été décrites (notamment dans BULL H.G. et al, (1985)J. Biol. Chem., 260, 2963-2972 ; HOOPER, N.M. et al, (1987) *Biochem.* J., 247, 85-93), et quelques séquences peptidiques de l'ECA endothéliale d'origine animale ont été publiées (BERNSTEIN, K.E. et al (1988), Kidney Int., 33, 652-655; HARRIS, R.B. et al (1985) J. Biol. Chem., 260, 2208-2211; IWATA, K. et al (1982) Biochem. Biophys. Res. Commun, 107, 1097-1103 ; IWATA, K. et al (1983) Arch. Biochem. Biophys., 227, 188-201 ; ST CLAIR, D.K. et al (1986) Biochem. Biophys. Res. Commun, 141, 968-972 ; SOFFER, RL. et al (1987) Clin. Exp. Hyp. A9, 229-234).

Quelques essais de clonage de l'ADN codant pour l'ECA animale ont été effectués à partir de deux organes riches en ECA, les reins et les poumons, mais aucun acide nucléique complet codant pour l'ECA animale n'a cependant été décrit ; seuls quelques fragments d'un tel acide nucléique ont été décrits (DELUCA-FLAHERTY, C. et al (1987) Int. J. Peptide Protein Res., 29, 678-684 ; BERNSTEIN K.E. et al, (1988), J. Biol. Chem., 263, 11021-11024). Les quantités d'ARN messager (ARNm) codant pour l'ECA sont probablement trop faibles dans ces organes pour permettre facilement le clonage d'un ADN complémentaire (ADNc) de cet ARNm.

Le clonage de l'ADN codant pour l'ECA des cellules endothéliales humaines a été réalisé pour la première fois par les auteurs de la présente demande ; la séquence nucléotidique complète de l'ADN codant pour l'ECA endothéliale, ainsi que la séquence en acides aminés de cette dernière sont décrits dans l'article de SOUBRIER F. et al paru dans Proc. Natl. Acad. Sci., USA, 85, pp 9386-9390 (1988).

Une activité enzymatique du type de celle de l'ECA a également été mise en évidence dans les testicules humains et animaux (J.J. LANZILLO et al, J. Biol. Chem., 260, pp 14938-14944 (1985)). Par ailleurs, Lanzillo et al. ont également rapporté l'identification de deux formes d'ECA dans les testicules humains (Biochem. Biophys. Res. Commun 12P, pp 457-63 (1985)). Lesdites formes présentaient des poids moléculaires de 140.000 et 90.000 Da et étaient respectivement présentes dans un rapport de 1 : 4 (Lanzillo et al. Chemical Abstracts. 102 (23), p.265 (1985), N°200077 s). Les deux formes de l'ECA testiculaire étaient similaires d'un point de vue catalytique, mais elles se distinguaient du point de vue immunologique.

Toutefois, la structure de l'ECA des testicules humains (ou encore désignée par l'expression ECA testiculaire) n'est pas connue à l'heure actuelle; et aucune séquence de l'ADN codant pour cette ECA testiculaire n'a été décrite jusqu'à maintenant. S.N. ROY et al affirment en 1988 dans un article de Biochem. Biophys. Res. Commun. 155 : 678-684, avoir isolé des clones mais ils ne donnent pas la séquence des clones.

La présente invention a précisément pour objet le clonage et le séquençage de l'acide nucléique codant pour l'ECA testiculaire humaine ; ce travail a été réalisé à partir de deux banques d'ADN complémentaires d'ARNm provenant de testicules humains et de sondes nucléotidiques correspondant à l'ECA endothéliale. Les techniques utilisées pour le clonage et le séquençage de l'acide nucléique selon l'invention seront plus particulièrement décrites dans la description détaillée de l'invention.

Il sera fait référence dans ce qui suit, aux figures dans lesquelles :
- la figure 1 représente l'acide nucléique ainsi que le polypeptide, déduit de ce dernier, correspondant à l'ECA testiculaire humaine ;
- la figure 2 représente les positions respectives des acides nucléiques des quatre clones utilisés pour la détermination de l'acide nucléique codant pour l'ECA testiculaire humaine.

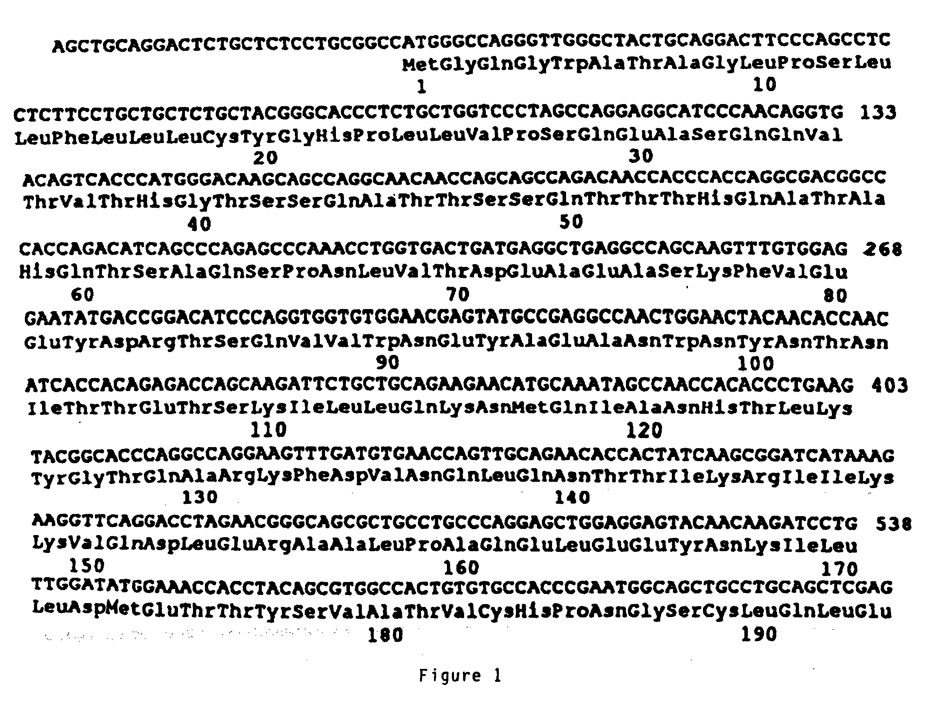

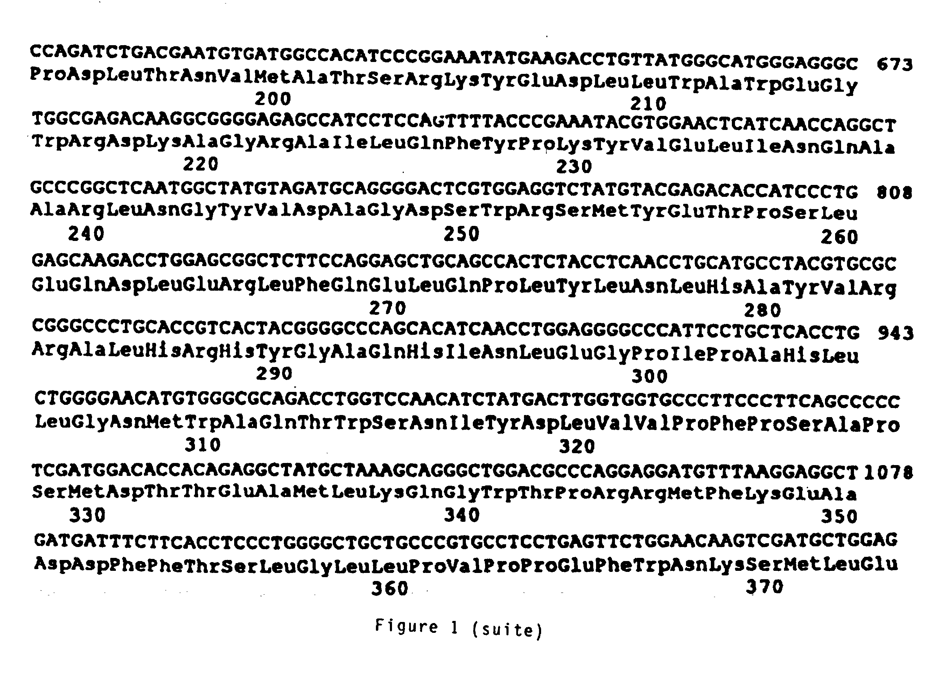

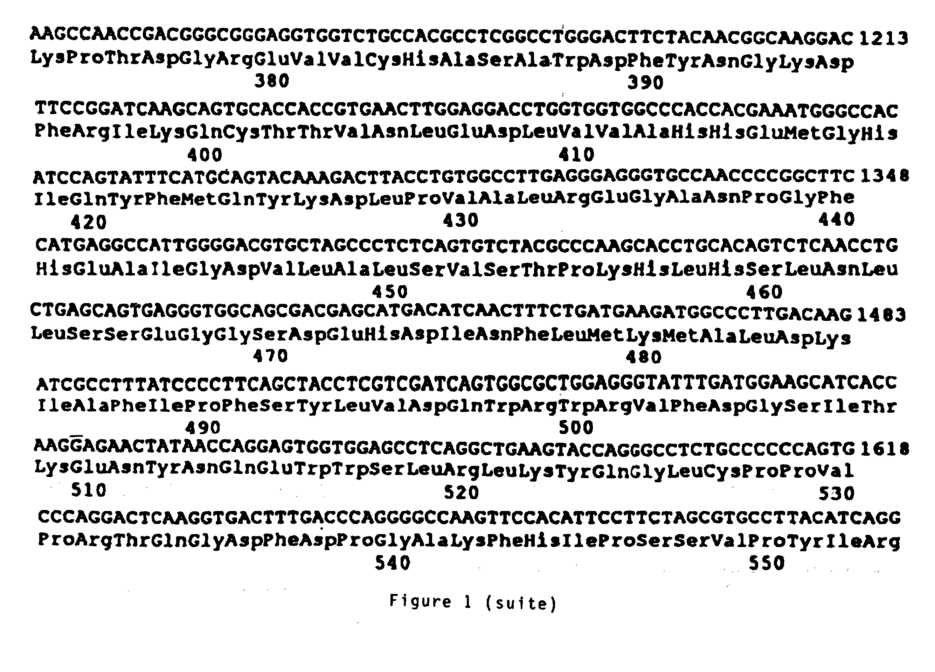

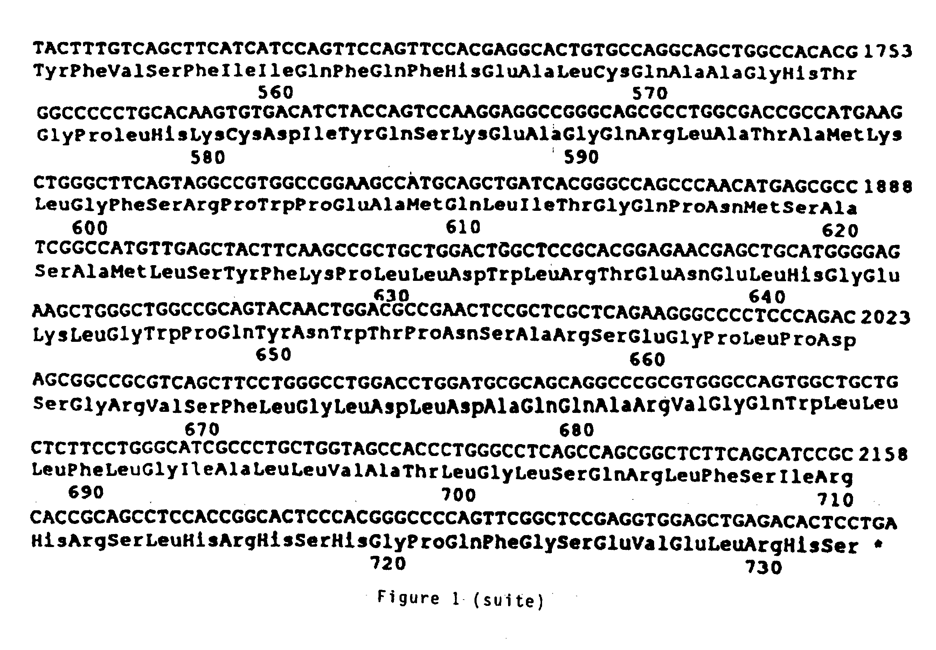

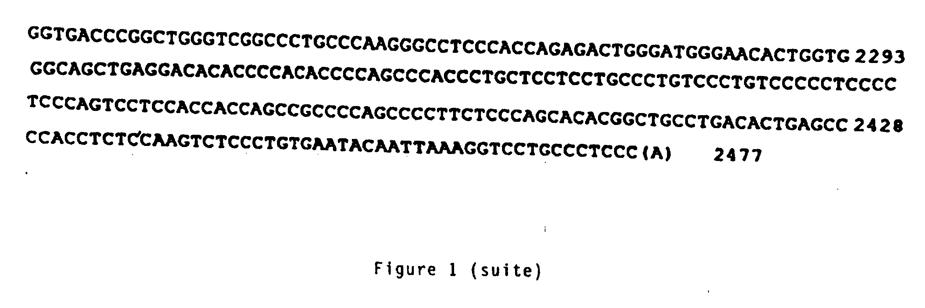

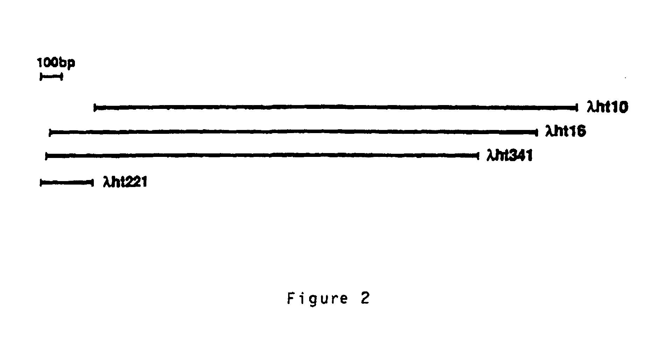

Une étude approfondie de l'acide nucléique de l'invention, ainsi que du polypeptide déduit à partir de ce dernier et correspondant à l'ECA testiculaire humaine, conduit aux observations suivantes :
- la séquence composite de l'ADNc de l'ACE testiculaire est de 2477 nucléotides, est délimitée par les nucléotides correspondant aux positions 1 et 2477 de la figure 1. La séquence nucléotidique, délimitée par les nucléotides situés aux positions 29 et 2224 de la figure 1, contient une phase de lecture ouverte codant pour un polypeptide de 732 acides aminés. Cette séquence nucléotidique est constituée d'une séquence d'ADN délimitée par les nucléotides correspondant aux positions 29 et 91 de la figure 1 susceptible de coder pour un peptide signal de 21 acides aminés, et d'une séquence d'ADN délimitée par les nucléotides situés aux positions 92 et 2224 de la figure 1 susceptible de coder pour une protéine mature de 711 acides aminés ;
- la séquence nucléotidique délimitée par les nucléotides situés aux positions 228 et 2224 de la figure 1 est identique à celle délimitée par les nucléotides situés aux positions 1944 à 3940 codant pour les 665 derniers acides aminés de l'ECA endothéliale humaine et représentée sur la figure 2 de l'article de SOUBRIER et al sus-mentionné ;
- la séquence nucléotidique délimitée par les nucléotides situés aux positions 29 et 229 codant pour les 67 premiers acides aminés de la pré-enzyme testiculaire (ou précurseur de l'enzyme testiculaire), et plus particulièrement la séquence délimitée par les nucléotides situés aux positions 92 et 229 codant pour pour les 46 premiers acides aminés de l'enzyme testiculaire mature, sont particulièrement spécifiques de l'ECA testiculaire ;
- le polypeptide comprenant une séquence His-Glu-Met-Gly-His correspondant aux acides aminés situés aux positions 414 à 418 de la figure 1, pourrait constituer une partie du site actif de l'ECA testiculaire.

La présente invention concerne donc tout acide nucléique caractérisé en ce qu'il comprend toute ou partie de la séquence nucléotidique de la figure 1, cette partie comprenant elle-même de 15 à 201 nucléotides de la séquence nucléotidique délimitée par les nucléotides situés aux positions 29 et 229 de la figure 1, et en ce qu'il code pour un polypeptide
- reconnu par des anticorps polyclonaux ou monoclonaux reconnaissant spécifiquement tout peptide issu de la séquence peptidique délimitée par les acides aminés situés aux positions 1 à 67 de la figure 1,
- et, le cas échant, capable d'hydrolyser l'angiotensine I et/ou les kinines, notamment la bradykinine. La présente demande décrit en outre un acide nucléique ne se distinguant du précédent au niveau de sa séquence nucléotidique que par des substitutions de nucléotides n'entraînant pas la modification de la séquence en acides aminés du susdit polypeptide dans des conditions propres à lui faire perdre les susdites propriétés.

Au sein de la séquence d'ADN délimitée par les nucléotides situés aux positions 29 et 2224 de la figure 1, et codant pour la pré-ECA testiculaire humaine (ou pro-enzyme) de 732 acides aminés, les 21 premiers acides aminés du côté N-terminal représentent le peptide signal, et les 711 acides aminés restant représentent l'ECA testiculaire humaine mature.

L'invention concerne également tout acide nucléique comprenant la séquence d'ADN délimitée par les nucléotides situés aux positions 92 et 2224 de la figure 1, et codant pour l'ECA testiculaire humaine mature.

Parmi les acides nucléiques conformes à l'invention, on citera notamment celui comprenant une séquence d'ADN s'étendant entre, d'une part, un nucléotide situé entre les positions 1 à 92 et, d'autre part, un nucléotide situé entre les positions 229 à 2224 de la figure 1.

L'invention a plus particulièrement pour objet tout acide nucléique comprenant la partie de la séquence nucléotidique de la figure 1 qui est spécifique de l'ECA testiculaire, à savoir tout acide nucléique contenant la séquence nucléotidique s'étendant entre, d'une part, le nucléotide situé à la position 29, et, d'autre part, le nucléotide situé à la position 229 de la figure 1, ou tout acide nucléique contenant une séquence issue de cette dernière.

A ce titre, l'invention concerne également :
- tout acide nucléique comprenant une séquence d'ADN d'environ 15, et de préférence 45, à 201 nucléotides, issue de la séquence nucléique s'étendant entre le nucléotide situé à la position 29 et le nucléotide situé à la position 229 de la figure 1,
- tout acide nucléique comprenant une séquence d'ADN d'environ 15, et de préférence 45, à 135 nucléotides, issue de la séquence nucléique s'étendant entre le nucléotide à la position 92 et le nucléotide situé à la position 229 de la figure 1.

La demande décrit aussi les acides nucléiques dérivés de ceux sus-mentionnés, et dont les séquences nucléotidiques sont modifiées dans les limites autorisées par la dégénérescence du code génétique, des lors que les polypeptides codés par ces acides nucléiques conservent soit une structure primaire identique, soit leurs propriétés immunologiques,et, le cas échéant enzymatiques.

De telles modifications non limitatives conduisent par exemple à des acides nucléiques variants qui se distinguent des acides nucléiques ci-dessus :
- par addition et/ou
- suppression d'un ou de plusieurs nucléotides et/ou
- modification d'un ou de plusieurs nucléotides.

L'invention a plus particulièrement pour objet tout acide nucléique présentant la caractéristique de s'hybrider spécifiquement avec la séquence délimitée par les nucléotides 29 à 229 de l'acide nucléique représenté sur la figure 1, notamment dans les conditions définies ci-après :
- dénaturation de l'acide nucléique (lorsqu'il est sous forme d'ADN double brin) susceptible de s'hybrider avec celui de la figure 1, avant sa fixation sur le support, défini ci-dessous, par traitement à l'aide d'une solution alcaline (0,5 M NaOH), suivie d'un passage à pH neutre.
- traitement de préhybridation du support (filtre de nitrocellulose ou membrane de nylon), sur lequel est fixé l'acide nucléique susceptible de s'hybrider avec celui de la figure 1, à 50°C pendant 3 heures avec une solution ayant la composition suivante : 25 mM KPO₄, pH 7,4 ; 5 x SSC ; 5 x Denhardt's ; 50 µg/ml d'ADN de sperme de saumon soniqué ; 0,1 % Sodium Dodecyl Sulfate (SDS ou NaDod SO₄⁻),
- remplacement de la solution de pré-hybridation au contact du support par une solution tampon ayant une composition identique à celle de pré-hybridation indiquée ci-dessus, (plus éventuellement 10 % de Dextran), et comprenant l'acide nucléique de la figure 1 en tant que sonde marquée, notamment de manière radioactive, et préalablement dénaturée par un traitement à 90°C pendant 3 minutes,
- incubation pendant 12 heures à 60°C,
- lavages successifs avec les solutions suivantes :
   . 2 x SSC, pendant 30 minutes à 60°C à 2 reprises,
   . 1 x SSC, 0,1 % SDS pendant 1 à 2 fois 15 minutes à 65°C.

Il est à rappeler que la composition de la solution de Denhardt est la suivante : 1 % ficoll, 1 % polyvinylpyrrolidone, 1 % BSA (albumine de sérum de boeuf), et que 1 x SSC est une solution 0,15 M de MaCl et 0,015 M de citrate de sodium, pH 7.

L'invention concerne également tout acide nucléique présentant la caractéristique de s'hybrider spécifiquement avec la séquence délimitée par les nucléotides 29 à 229 de l'acide nucléique de la figure 1 dans des conditions non stringentes reprenant les caractéristiques essentielles des conditions stringentes définies ci-dessus, sauf pour ce qui concerne la température qui est de 40°C dans les conditions non stringentes, et les lavages successifs qui, dans les conditions non stringentes, sont réalisés à 1 'aide de 2 x SSC avec ou sans SDS à 45°C pendant 15 minutes à 2 reprises, et présentant en outre la caractéristique de coder pour un polypeptide, ce polypeptide étant :
- reconnu par des anticorps polyclonaux ou monoclonaux reconnaissant spécifiquement la séquence peptidique délimitée par les acides aminés situés aux positions 1 à 67 de la figure 1,
- et, le cas échéant, capable d'hydrolyser l'angiotensine I et/ou les kinines, notamment la bradykinine.

La présence demande décrit aussi tout acide nucléique susceptible de s'hybrider avec les acides nucléiques issus de celui de la figure 1 tels que définis ci-dessus, ou leurs séquences complémentaires. notamment dans les conditions d'hybridation décrites ci-dessus.

L'invention vise également les sondes nucléotidiques susceptibles de s'hybrider avec tout fragment de l'acide nucléique de la figure 1 tel que défini ci-dessus, ainsi qu'avec l'ARN messager codant pour l'ECA testiculaire et avec le gène humain responsable de l'expression de l'ECA testiculaire, notamment dans les conditions d'hybridation définies ci-dessus, ou leurs séquences complémentaires.

Il va de soi que les conditions d'hybridation stringentes ou non stringentes, définies ci-dessus constituent des conditions préférées pour l'hybridation, mais ne sont nullement limitatives et peuvent être modifiées sans affecter pour autant les propriétés de reconnaissance et d'hybridation des sondes et des acides nucléiques sus-mentionnés.

Les conditions salines et de température, au cours de l'hybridation et du lavage des membranes, peuvent être modifiées dans le sens d'une plus grande ou d'une plus faible stringence sans que la détection de l'hybridation en soit affectée.. Par exemple, il est possible d'ajouter un pourcentage variable de formamide pour abaisser la température au cours de l'hybridation.

L'invention décrit également le polypeptide de la figure 1 correspondant à l'ECA testiculaire humaine, ainsi que tous les polypeptides, codés par les fragments d'ADN sus-mentionnés issus de l'acide nucléique de la figure 1, susceptibles d'être reconnus par des anticorps polyclonaux ou monoclonaux reconnaissant spécifiquement tout peptide issu de la séquence peptidique délimitée par les acides aminés situés aux positions 1 à 67 de la figure 1, et plus particulièrement celle délimitée par les acides aminés situés aux positions 22 à 67 de la figure 1, et, le cas échéant, de posséder une activité enzymatique du type de celle de l'ECA testiculaire

Parmi les polypeptides sus-mentionnés décrits dans la demande, on distinguera notamment :
- le polypeptide s'étendant entre les acides aminés correspondant aux positions 1 et 732 de la figure 1 ;
- le polypeptide s'étendant entre les acides aminés correspondant aux positions 22 et 732 et les peptides suivants, objet de l'invention:
   - le polypeptide dont la séquence peptidique est délimitée par les acides aminés situés aux positions 1 et 67 de la figure 1,
   - le polypeptide dont la séquence peptidique est délimitée par les acides aminés situés aux positions 22 et 67 de la figure 1,
   - le polypeptide caractérisé par la séquence peptidique d'environ 5 à 67 acides aminés, issue de la séquence peptidique délimitée par les acides aminés situés aux positions 1 et 67 de la figure 1,
   - le polypeptide caractérisé par la séquence peptidique d'environ 5 à 45 acides aminés, issue de la séquence peptidique délimitée par les acides aminés situés aux positions 22 et 67 de la figure 1.

Les polypeptides précédents peuvent être modifiés dès lors qu'ils conservent les propriétés biochimiques ou immunologiques ou pharmacologiques définies précédemment.

Par exemple, et de façon non limitative, des polypeptides dans le cadre de l'invention peuvent se distinguer des polypeptides définis ci-dessus ;
- par addition et/ou
- suppression d'un ou plusieurs acides aminés et/ou
- modification d'un ou de plusieurs acides aminés, sous réserve que les propriétés biochimiques ou immunologiques ou pharmacologiques comme indiqué ci-dessus soient conservées.

En l'occurrence, l'invention concerne un polypeptide tel que ceux mentionnés précédemment, ledit polypeptide étant couplé à la sérum-albumine bovine, à la KLH ou à la LPH (voir infra).

On conçoit que l'homme de métier dispose de la possibilité d'identifier, voire de sélectionner ceux des polypeptides de séquences plus courtes qui entrent dans le champ de l'invention. A titre de l'un des moyens généraux lui permettant de procéder à cette identification, on mentionnera, par exemple, le traitement du polypeptide de la figure 1 avec une protéase clivant le polypeptide sus-mentionné dans un site peptidique choisi, soit dans une région N-terminale, soit dans une région C-terminale, suivi de la séparation du fragment N-terminal ou du fragment C-terminal du restant dudit polypeptide, ce "restant" étant alors testé pour ses propriétés de reconnaissance par un anticorps monoclonal ou polyclonal dirigé contre un peptide issu de la séquence peptidique délimitée par les acides aminés situés aux positions 22 à 67 de la figure 1, et, le cas échéant, pour son activité enzymatique vis-à-vis de l'angiotensine et/ou de la bradykinine. En cas de réponse positive, l'on aura alors établi que le fragment N-terminal ou C-terminal, ne jouait pas un rôle significatif, sinon essentiel pour la manifestation des propriétés immunologiques et/ou enzymatiques dudit polypeptide. L'opération peut éventuellement être répétée pour autant que l'on dispose d'une protéase susceptible de reconnaître un autre site spécifique proche d'une extrémité N-terminale ou C-terminale du polypeptide restant. La perte par le polypeptide plus petit reconnu des propriétés immunologiques et/ou enzymatiques du fragment plus long dont il était issu peut conduire à l'hypothèse que le dernier fragment séparé jouait un rôle significatif dans la manifestation des propriétés enzymatiques du polypeptide de la figure 1.

Une autre variante, plus simple que la précédente, de détection des régions de l'ECA testiculaire essentielles à la manifestation des activités enzymatiques de cette dernière, peut être basée sur des traitements enzymatiques d'un acide nucléique codant pour l'ECA testiculaire. Ce traitement est réalisé avant l'incorporation de l'acide nucléique ainsi obtenu, et présumé coder pour un polypeptide possédant des activités immunologiques et/ou enzymatiques du type de celle de l'ECA testiculaire, dans le vecteur d'expression utilisé pour la mise en oeuvre d'un procédé de production dudit polypeptide dans l'hôte cellulaire approprié (procédé qui sera plus particulièrement détaillé dans ce qui suit). Ce traitement enzymatique peut alors consister soit en un émondage des extrémités de l'acide nucléique initial (codant par exemple pour le polypeptide de la figure 1 en entier), par exemple par une enzyme exonucléolytique, telle que Ba131, soit par une ou plusieurs enzymes de restriction choisie pour leurs sites de reconnaissance respectifs (le cas échéant aménagés par mutagenèse ponctuelle dirigée) dans la séquence de l'acide nucléique initial, soit par l'addition d'un fragment d'ADN synthétique de jonction entre le site de coupure de l'enzyme de restriction et le début de la région à exprimer. On peut ainsi supprimer à partir de l'extrémité 3' de l'ARN messager, des séquences de longueurs croissantes, remplacées par un codon stop de traduction. La même démarche peut s'appliquer à l'extrémité 5' mais nécessite de maintenir un codon d'initiation (ATG), le signal peptide ou tout autre séquence permettant la sécrétion du peptide et de maintenir la phase ouverte de lecture.

L'acide nucléique tronqué obtenu peut alors être testé, après incorporation dans le vecteur choisi et transformation de l'hôte cellulaire avec le vecteur recombinant obtenu, pour sa capacité à exprimer un polypeptide tronqué correspondant, possédant encore les susdites propriétés immunologiques et/ou enzymatiques ou au contraire ne les possédant plus, d'où la possibilité, comme dans la variante précédente, d'identifier au sein du polypeptide de la figure 1, les séquences jouant un rôle important, sinon essentiel dans la manifestation des propriétés immunologiques et/ou enzymatiques de l'ECA testiculaire.

L'invention a également pour objet tout acide nucléique recombinant contenant tout fragment d'ADN du type sus-indiqué codant pour la pré-ECA testiculaire humaine, ou l'ECA testiculaire humaine mature, ou encore pour tout polypeptide, susceptible de posséder une activité immunologique et/ou enzymatique du type de celle de l'ECA testiculaire, associé avec un promoteur et/ou un terminateur de transcription reconnu par les polymérases de la cellule hôte dans laquelle ledit acide nucléique recombinant est susceptible d'être introduit.

L'introduction dudit acide nucléique recombinant dans la cellule hôte, est avantageusement réalisée à l'aide de vecteurs, notamment du type plasmide, qui sont aptes à se répliquer dans ladite cellule hôte et à y permettre l'expression de la séquence codant pour l'enzyme.

Ledit acide nucléique recombinant peut également être introduit dans la cellule hôte à l'aide d'un vecteur viral (virus recombinant) capable d'infecter ladite cellule hôte, ou à l'aide d'un vecteur rétroviral capable de s'intégrer dans le génome de la cellule hôte, et d'y permettre l'expression du polypeptide codé par un acide nucléique selon l'invention, ce dernier étant sous le contrôle d'un promoteur viral, ou rétroviral, actif dans la cellule hôte.

A titre d'exemples de vecteurs utilisables dans le cadre de la présente invention, on citera le virus de la leucémie murine de type Moloney (ou M-MuLV) susceptible d'infecter les cellules NIH 3T3, ou le baculovirus utilisé pour infecter les cellules d'insectes.

L'invention concerne donc un procédé de production de l'ECA testiculaire humaine, ou des polypeptides sus-mentionnés issus de l'ECA testiculaire, qui comprend la transformation des cellules hôtes au moyen des vecteurs sus-indiqués, la mise en culture des cellules hôtes transformées dans un milieu approprié et la récupération desdits polypeptides soit directement à partir du milieu de culture, lorsque ces derniers y sont secrétés (notamment dans le cas où les polypeptides considérés sont précédés d'une séquence signal lors de leur synthèse dans la cellule hôte), soit après lyse de la paroi de la cellule hôte dans le cas où les polypeptides ne seraient pas secrétés hors de cette dernière.

Les cellules hôtes utilisées pour la mise en oeuvre du procédé sus-mentionné peuvent être des cellules procaryotes, notamment des cellules de E. coli, ou, d'une manière plus avantageuse, des cellules eucaryotes, qui permettent, notamment, d'obtenir des protéines sous leur forme glycosylée et mature (levures, cellules CHO ou autres cellules, ou cellules d'insectes infectées par le baculovirus).

L'invention vise également un procédé de préparation des nouveaux polypeptides sus-mentionnés par synthèse qui comprend soit l'addition étape par étape des résidus peptidiques choisis, avec l'addition ou l'élimination de groupes protecteurs quelconques des fonctions amino et carboxyle, ou addition de résidus peptidiques choisis afin de produire des fragments, suivie d'une condensation desdits fragments en une séquence en acides aminés appropriée, avec addition ou élimination des groupes protecteurs choisis.

L'invention se rapporte également à des anticorps spécifiques dirigés contre les polypeptides ci-dessus. En particulier, l'invention vise des anticorps polyclonaux ou monoclonaux dirigés contre les séquences peptidiques sus-mentionnées.

Un mode d'obtention d'anticorps polyclonaux contre un peptide dérivé de la séquence de l'ECA testiculaire est brièvement résumé ci-après :
- le peptide dont la taille minimum est d'environ 5 à 15 acides aminés, est couplé
   * soit à la sérum albumine bovine ou à la KLH (hemocyanine de Keyhole Limpets (Megathura crenulata) si le peptide contient une cystéine, qui peut être ajoutée de façon artificielle à la séquence naturelle. Cette cystéine sera auparavant activée par le SPDP = Ester N-Hydroxysuccinimide de l'acide (pyridyldithio-2)-3 propionique,
   * soit à la KLH ou à la LPH (hemocyanine de l'hemolymphe de Limulus polyhemas) par l'intermédiaire de la benzoquinone;
   * soit à la LPH par le glutaraldéhyde,
   * soit à la LPH par le carbodiimide,
- le peptide couplé est ensuite injecté au lapin dans un volume égal d'adjuvant de Freund et dans le coussinet plantaire des pattes postérieures des lapins. Lors de la première injection, c'est de l'adjuvant de Freund complet qui est utilisé. Ensuite c'est de l'adjuvant incomplet,
- une injection de rappel est pratiquée toutes les 3 à 4 semaines,
- entre chaque injection, un prélèvement de sang est effectuée pour mesurer le titre des anticorps (le titre des anticorps est établi en mesurant la dilution pour laquelle le peptide qui a servi aux injections et qui est marqué radioactivement est lié à l'anticorps à 50%).

Les anticorps monoclonaux sus-mentionnés peuvent être produits par la technique des hybridomes dont le principe général est rappelé ci-après.

Dans un premier temps, on inocule un des polypeptides ci-dessus à un animal choisi (par exemple souris de type Biozzi ou Balb/C), dont les lymphocytes B sont alors capables de produire des anticorps contre ce polypeptide. Le protocole d'injection comprend par exemple trois injections dont la première faite avec de l'adjuvant de Freund complet. Les animaux peuvent recevoir une injection I.V. du polypeptide quelques heures avant l'isolement des cellules de la rate. Les cellules spléniques de l'animal injecté sont ensuite fusionnées avec les cellules "immortelles" de la lignée myélomateuse, selon la méthode décrite par DI PAULI (DI PAULI R, and W.C., RASCHKE, (1978), in Current topics in Microbiology and Immunology, vol. 81,. F. MELCHERS, M. POTTE, and N.L. WARNER, editors, Springer-Verlag, Berlin, 37-39), à l'aide du polyéthylène glycol. Les cellules ayant fusionnées (hybridomes) sont ensuite remises en culture et sélectionnées dans un milieu hypoxanthineaminoptérine-thymidine. A partir du mélange hétérogène des cellules ainsi obtenu, on réalise alors une sélection des cellules capables de produire un anticorps particulier et de se multiplier indéfiniment.

Les hybridomes produisant des anticorps sont ensuite clonés et sous-clonés, au mieux grâce à un trieur de cellules. Les hybridomes sont ensuite réinjectés dans le péritoine d'animaux, notamment de souris Balb/C traitées par le pristane. Les ascites sont ensuites testées pour la présence d'anticorps et les immunoglobulines présentes dans les ascites sont purifiées sur colonne d'affinité, notamment sur protéine-A Sépharose.

Des anticorps mono ou polyclonaux peuvent également être produits contre la molécule entière d'ECA.

Parmi les polypeptides utilisés pour la fabrication des anticorps polyclonaux ou monoclonaux sus-mentionnés, on mentionnera plus particulièrement les polypeptides spécifiques de l'ECA testiculaire à savoir ceux délimités par les acides aminés situés aux positions 1 et 67, ou 22 et 67 de la figure 1, ou encore ceux possédant au moins 5 acides aminés issus de la séquence peptidique délimitée par les acides aminés situés aux positions 1 et 67 de la figure 1.

L'invention concerne également une méthode de dépistage, ou de dosage, in vitro d'ECA ou produit ayant in vivo les propriétés de l'ECA, et plus particulièrement de l'ECA testiculaire humaine, ou d'un produit dérivé de l'ECA testiculaire tel que les polypeptides sus-mentionnés, dans un prélèvement biologique susceptible de les contenir. Une telle méthode de dépistage selon l'invention, peut être réalisée soit à l'aide des anticorps monoclonaux susmentionnés, soit à l'aide des sondes nucléotidiques décrites ci-dessus.

L'invention a plus particulièrement pour objet l'utilisation des anticorps polyclonaux ou monoclonaux obtenus à partir du polypeptide délimité par les acides aminés situés aux positions 1 et 67, ou celui délimité par les acides aminés situés aux positions 22 et 67, ou encore celui issu de la séquence peptidique délimitée par les acides aminés situés aux positions 1 et 67 de la figure 1, et reconnaissant spécifiquement ces polypeptides, pour la mise en oeuvre d'une méthode de dépistage, ou de dosage, in vitro, de l'ECA testiculaire humaine.

L'invention concerne également l'utilisation des sondes nucléotidiques décrites ci-dessus, notamment celles s'hybridant avec les séquences nucléiques codant pour les polypeptides spécifiques de l'ECA testiculaire humaine décrits ci-dessus, pour la mise en oeuvre de la méthode de dépistage ou de dosage sus-mentionnée.

Le prélèvement biologique sus-mentionné est effectué soit dans des tissus fluides, tels que le sang, soit dans des organes, ce dernier type de prélèvement permettant notamment d'obtenir des coupes fines de tissus sur lesquelles les anticorps ou les sondes sus-mentionnés sont ultérieurement fixés.

La méthode de dosage selon l'invention, procédant par l'intermédiaire des anticorps sus-mentionnés comprend notamment les étapes suivantes :
- la mise en contact d'un anticorps reconnaissant spécifiquement l'ECA testiculaire ou un polypeptide dérivé de l'ECA testiculaire selon l'invention, avec le susdit prélèvement biologique dans des conditions permettant la production éventuelle d'un complexe immunologique formé entre l'ECA testiculaire ou produit qui en est dérivé et l'anticorps sus-mentionné;
- la détection à l'aide de tout moyen approprié du susdit complexe immunologique.

Avantageusement, les anticorps utilisés pour la mise en oeuvre d'un tel procédé sont marqués notamment de manière enzymatique ou radio-active.

Une telle méthode selon l'invention peut notamment être réalisée suivant la méthode ELISA (enzyme linked sorbent assay) qui comprend les étapes suivantes :
- fixation d'une quantité prédéterminée d'anticorps sur un support solide, notamment à la surface d'un puits d'une microplaque ;
- addition du prélèvement biologique (sous forme liquide) sur ledit support ;
- incubation pendant une temps suffisant pour permettre la réaction immunologique entre lesdits anticorps et l'ECA testiculaire ou produit qui en est dérivé sus-mentionné ;
- élimination des parties non fixées du prélèvement biologique et lavage du support solide (en particulier des puits de la microplaque) ;
- addition d'une immunoglobuline marquée par une enzyme susceptible d'activer un substrat spécifique de cette enzyme,
- addition du substrat spécifique de l'activité enzymatique libérée lors de la réaction immunologique précédente ;
- détection, à l'aide de tout moyen approprié, de la dégradation du substrat par l'enzyme ; et
- corrélation de la quantité d'enzymes libérées à la concentration d'ECA humaine initialement présente dans le prélèvement biologique.

Selon un autre mode de réalisation de la méthode de dosage de l'invention, les anticorps sus-mentionnés ne sont pas marqués et la détection des complexes immunologiques formés entre les polypeptides et lesdits anticorps est réalisée à l'aide d'une immunoglobuline marquée reconnaissant lesdits complexes.

La méthode de dosage selon l'invention peut également être réalisée par une technique immunoenzymatique suivant un mécanisme de compétition entre les polypeptides susceptibles d'être contenus dans le prélèvement biologique, et des quantités prédéterminées de ces mêmes polypeptides, vis-à-vis des anticorps sus-mentionnés. Dans ce dernier cas, les polypeptides de l'invention en quantité prédéterminée sont avantageusement marqués à l'aide d'un marqueur enzymatique.

L'invention ne se limite nullement aux modes de réalisation décrits ci-dessus pour le dosage in vitro des polypeptides de l'invention, ce dosage pouvant être réalisé à l'aide de toute autre méthode immunologique appropriée.

L'invention concerne également une méthode de dépistage, ou de dosage, in vitro d'une ECA ou produit ayant in vivo les propriétés de l'ECA, et, plus particulièrement, de l'ECA testiculaire humaine, cette méthode procédant par détection ou dosage d'un acide nucléique correspondant selon le code génétique universel à toute ou partie de cette ECA, et étant réalisée à partir d'un prélèvement biologique susceptible de contenir ledit acide nucléique. Cette méthode est caractérisée en ce qu'elle comprend :
- la mise en contact d'une sonde nucléotidique décrite ci-dessus avec le susdit prélèvement biologique dans des conditions permettant la production éventuelle d'un complexe d'hybridation formé entre l'acide nucléique à détecter et ladite sonde ;
- la détection à l'aide de tout moyen approprié du susdit complexe d'hybridation.

Selon un mode de réalisation de l'invention, le prélèvement biologique sus-mentionné est, préalablement à la mise en oeuvre du dépistage, traité de manière à ce que les cellules qu'il contient soient lysées et, éventuellement, en ce que le matériel génomique contenu dans lesdites cellules soit fragmenté à l'aide d'enzymes de restriction du type EcoRI, BamHI etc..., ou que les ARN en soient isolés, notamment selon la méthode décrite dans THOMAS, P.S. (1983) Methods Enzymol., 100, 255-266.

Avantageusement, les sondes nucléotidiques de l'invention sont marquées, notamment par l'incorporation d'un (ou plusieurs) nucléotide soit radio-actif soit couplé à un haptène permettant sa détection par un anticorps, ce dernier étant conjugué à une enzyme dont l'activité est facilement détectable (par exemple la phosphatase alcaline). Les ADN, ou ARN, issus du prélèvement biologique sont placés sur un support approprié, notamment sur un filtre de nitrocellulose ou autre, par exemple membrane de nylon, sur lequel sont ensuite additionnées les sondes sus-mentionnées.

Selon un autre mode avantageux de réalisation du procédé sus-mentionné de l'invention, des coupes histologiques sont réalisées à partir du susdit prélèvement biologique et les sondes nucléotidiques de l'invention sont mises en contact direct avec des coupes histologiques pour la détection des acides nucléiques de l'invention par hybridation in situ.

L'invention concerne également l'application de la méthode de dépistage ou de dosage de l'ECA testiculaire sus-mentionnée aux diagnostic in vitro de pathologies spécifiques des testicules (en particulier au niveau des cellules germinales), notamment de tumeurs de testicules (encore appelés séminomes), de la maturation des cellules germinales mâles, des hypofertilités et stérilités masculines.

Dans le cadre de diagnostic de l'hypofertilité et de la stérilité masculine, la méthode sus-mentionnée est avantageusement réalisée à partir du sperme, ou de cellules germinales ou du plasma séminal isolés à partir du sperme, ou à partir de biopsie testiculaire.

L'invention a également pour objet des nécessaires ou kits pour la mise en oeuvre des méthodes de dépistage, ou de dosage, in vitro susmentionnés.

A titre d'exemple, de tels kits comprennent notamment:
- une quantité déterminée d'un des anticorps polyclonaux ou monoclonaux sus-mentionnés susceptible de donner lieu à une réaction immunologique spécifique avec l'ECA testiculaire ou avec un des polypeptides dérivés de l'ECA testiculaire selon l'invention ;
- et/ou une quantité déterminée d'ECA testiculaire ou un polypeptide susceptible de donner lieu à une réaction immunologique avec les anticorps sus-mentionnés ;
- avantageusement, un milieu approprié à la formation d'une réaction immunologique entre l'ECA ou les polypeptides de l'invention et les anticorps susmentionnés ;
- avantageusement, des réactifs permettant la détection des complexes immunologiques produits lors de la réaction immunologique sus-mentionnée.

Dans le cadre de la mise en oeuvre d'une méthode de dépistage in vitro utilisant des sondes nucléotidiques, les kits utilisés comprennent par exemple :
- une quantité déterminée d'une des sondes nucléotidiques sus-mentionnées susceptible de donner lieu à une réaction d'hybridation avec un des acides nucléiques sus-mentionnés codant pour l'ECA testiculaire ou un polypeptide dérivé de l'ECA testiculaire selon l'invention ;
- avantageusement, des réactifs permettant la détection des complexes d'hybridation produits lors de la réaction d'hybridation sus-mentionnée.

L'invention concerne également l'utilisation du polypeptide de la figure 1 ou de tout fragment peptidique approprié issu de ce dernier, pour la conception de nouveaux inhibiteurs de l'ECA humaine, plus puissants et/ou spécifiques de cette dernière que ne le sont les actuels inhibiteurs de l'ECA.

L'invention a également pour objet une méthode de détection ou de dosage, d'un inhibiteur de l'ECA, ou de quantification de son pouvoir inhibiteur, qui comprend la mise en contact du polypeptide de la figure 1, ou de tout fragment peptidique issu de ce dernier et possédant une activité enzymatique du type de celle de l'ECA, avec ledit inhibiteur, et la détermination, pour ces inhibiteurs de la constante de dissociation [Ki] et de la concentration nécessaire à un inhibition de 50% [IC₅₀]de l'enzyme.

L'invention concerne également l'utilisation du polypeptide de la figure 1, ou de tout fragment de ce dernier, tel que décrit ci-dessus, capable d'hydrolyser les kinines, notamment la bradykinine, dans le traitement des maladies inflammatoires, ou infectieuses, de la pancréatite aigue, et plus généralement de maladies où une libération de kinines dans l'organisme pourrait jouer un rôle pathogène.

A ce titre, l'invention concerne plus particulièrement des compositions pharmaceutiques pour le traitement des maladies indiquées ci-dessus, caractérisée par l'association de tout ou partie du polypeptide de la figure 1, capable d'hydrolyser les kinines, notamment la bradykinine, avec un véhicule pharmaceutiquement acceptable.

La présente invention a également pour objet l'utilisation des sondes nucléotidiques de l'invention, capables de s'hybrider avec le gène responsable de l'expression de l'ECA testiculaire humaine dans les conditions décrites ci-dessus, pour la détermination des différentes formes alléliques du gène sus-mentionné.

L'invention a également pour objet toute séquence nucléotidique d'environ 15 à environ 30 nucléotides issue de la séquence nucléotidique de la figure 1, ou une séquence complémentaire de cette dernière, ou encore une séquence d'environ 15 à 30 nucléotides susceptible de s'hybrider (dans les conditions décrites ci-dessus) avec ladite séquence d'environ 15 à 30 nucléotides issue de la figure 1, et l'utilisation de telles séquences en tant qu'amorces permettant l'amplification génique de toute ou partie de la séquence nucléotidique de la figure 1, notamment selon la méthode d'amplification décrite dans la demande de brevet européen n° 86/302.298.4 du 27/03/1986.

Ces amorces oligonucléotidiques sont particulièrement utiles dans le cadre de la synthèse de l'ECA testiculaire par voie du génie génétique telle que décrite ci-dessus, dont les différentes étapes sus-mentionnées sont précédées par une étape d'amplification du gène codant pour l'ECA testiculaire préalablement à l'étape de transformation des cellules hôtes.

L'invention concerne également une méthode de détection in vitro de l'ARNm codant pour l'ECA testiculaire, telle que décrite ci-dessus, comprenant une étape préalable d'amplification de toute ou partie de cet ARNm (ou l'amplification de la copie d'ADN de l'ARN codant pour l'ECA testiculaire), le cas échéant, isolé des autres constituants cellulaires, à l'aide d'un ou de plusieurs, couple d'amorces telles que définies ci-dessus.

A ce titre, l'invention a également pour objet des kits de diagnostic tels que décrits ci-dessus, et contenant également au moins un couple d'amorces sus-mentionnées, ces amorces étant susceptibles d'amplifier le nombre de copies de l'acide nucléique à détecter en présence d'une polymérase appropriée et des quantités appropriées des 4 désoxynucléotides triphosphate différents, dATP, dGTP, dCTP et dTTP.

Des caractéristiques supplémentaires de l'invention apparaîtront encore au cours de la description qui suit du clonage de l'acide nucléique codant pour l'ECA testiculaire humaine, ainsi que des sondes utilisées pour ce clonage, étant entendu que cette description ne saurait être interprétée comme tendant à restreindre la portée des revendications.

### I - SEQUENCAGE DE L'ADN COMPLEMENTAIRE DE L'ECA TESTICULAIRE HUMAINE

Une banque d'ADNc de testicules humains amorcée avec de l'oligo(dT), construite dans le phage λgt11 (clontech laboratories Inc.) a été criblée en utilisant la sonde décrite ci-dessous.

Les hybridations avec les phages ont été effectuées selon la méthode de Benton WD et Davis RW Science (WASHINGTON) 1977, 196, 180-182. Plus précisément les hybridations ont été effectuées dans une solution constituée de 6XSSC (1XSSC correspond à NaCl 150 mM, citrate de sodium 15 mM), 0,1 % de NaDodSO₄, tampon NaPO₄ 50 mM à pH 6,8, 0,1 mg/ml d'ADN dénaturé, de sperme de saumon à 65°C.

La séquence utilisée comme sonde est contenue dans le bactériophage λ19-22 et représente les 3248 derniers nucléotides de la séquence d'ADN complémentaire (position 691 à 4024), telle que décrite dans l'article de SOUBRIER F. et al, sus-mentionné. Elle a été choisie car elle hybride avec l'ARN messager de l'ECA dans le testicule humain. La séquence clonée de l'ADN de l'ECA endothéliale a été utilisée comme sonde, par marquage statistique à l'aide de la DNA polymérase I de E.coli et d'amorces oligonucléotidiques, selon la méthode décrite par FEINBERG AP et al (1983) Anal. Biochem., 132, 6-13. En utilisant l'alpha [³²P], deoxycytidine triphosphate, d'activité spécifique 3000cies/mmole, la sonde a une activité spécifique de 2 à 8 x 10⁹ cpm/µg d'ADN. La concentration de la sonde radioactive dans la solution d'hybridation des filtres est de 10⁶ cpm/ml. Les filtres ont ensuite été lavés en condition de forte stringence (lavage final en 0,1 x SSC, 0,1 % NaDodSO₄) à 68°C pendant 30 minutes. Le criblage de 2 x 10⁶ clones de la banque a permis d'isoler 17 clones positifs. Parmi ceux-ci , deux clones positifs ont été isolés. Les fragments d'ADN insérés dans les phages recombinants ont été purifiés et insérés dans le site EcoRI du plasmide pBluescript (Sratagene). La séquence des insertions a été obtenue soit directement sur l'ADN double brin des plasmides, soit en obtenant de l'ADN simple brin en infectant les cultures à l'aide du phage helper KO7.

La détermination de la séquence nucléotidique de ces clones a été effectuée par la méthode de Sanger (SANGER, F. et al (1977), Proc. Natl. Acad. Sci., USA, 74, 5463-5467) en utilisant soit l'ADN polymérase de klenow, soit l'ADN polymérase de T7 modifiée (Sequenase, US Biochemical). Quelques régions ont été séquencées en utilisant à la place de la déoxyguanosine tri-phosphate (dGTP), le déoxyinosine tri-phosphate (dITP) ou le deaza-déoxyguanosine triphosphate (7-deaza dGTP). L'électrophorèse des fragments marqués par le [S³⁵] dATP a été pratiquée sur gel d'urée-polyacrylamide. Les séquences ont été déterminées en synthétisant des oligomères de proche en proche toutes les 350 paires de base environ qui ont servi d'amorces échelonnées le long de la séquence.

Le clone λht10 contient une séquence d'une longueur de 2217 nucléotides et qui s'étend de la position 260 à 2477 de la séquence du cDNA testiculaire portée sur la figure 1. Ce clone s'étend dans la région 3' de l'ARN messager jusqu'à la séquence de polyadénylation et comprend une courte séquence poly(A). Le clone λht16, d'une longueur de 2263 nucléotides, correspond à la région du cDNA testiculaire située entre la position 50 et 2313 de la figure 1. Un oligomer d'une longueur de 22 nucléotides, a été synthétisé et utilisé pour cribler à nouveau la banque afin d'obtenir des clones correspondant à la région la plus 5' de l'ARN messager. La séquence de cet oligomer est contenue dans la séquence du clone λht16 (position 59 à 80). L'oligomer a été marqué auN γ-[³²P-] adénosine triphosphate ATP (activité spécifique 5000cies/mmole) à l'aide de la T4 polynucléotide kinase. L'activité spécifique de la sonde est de 5 x 10⁶ cpm/pmole. Les filtres ont été criblés dans les mêmes conditions que précisées au dessus à part les modification suivantes : la température d'hybridation était de 60°C et la température de lavage de 65°C dans une solution 2 X SSC. Ce criblage a permis d'isoler le clone λht341, d'une longueur de 2,2 kb, qui s'étend en 5' jusqu'au nucléotide 31 du cDNA testiculaire de l'ACE.

De manière à obtenir l'ADN complémentaire correspondant à l'extrémité 5' de l'ARN messager codant pour l'ECA testiculaire, une autre banque de donnée d'ADNc a été construite en utilisant 5 µg d'ARN isolé à partir de tissu testiculaire humain obtenu lors d'une intervention chirurgicale. La banque d'ADN complémentaire a été construite selon la méthode de Gübler et Hoffman (Gene (1983), 25, 263-269), en utilisant deux amorces. D'une part une amorce spécifique de l'ARN messager de l'ECA, déterminé par la séquence des clones précédemment obtenus. Il s'agit d'un oligomer de 17 bases (ATH17), complémentaire d'une séquence localisée en position 228-244 de la figure 1. La seconde amorce correspond à l'oligo-d(T)12-18 mers (pharmacia). Les ADN complémentaires double-brin ont été synthétisés puis insérés dans le phage λgt10 coupé par l'enzyme EcoRI selon la méthode de Koenig et al (Koenig, M et al,(1987) Cell, 50, 509-517).

Les phages recombinants (1 x 10⁶ phages recombinants) ont été criblés à l'aide de l'oligomer TH16, marqué comme précédemment. Les conditions de criblage de la banque sont identiques à celles décrites plus haut avec la même sonde. Parmi les clones positifs obtenus, le clone λht221, d'une longueur de 244 paires de base a été séquence. Il contient la partie la plus 5' de l'ARN messager codant pour l'ECA testiculaire.

La séquence de l'ARN messager testiculaire, telle qu'elle peut être déduite de la séquence nucléotidique composite du cDNA testiculaire obtenue à partir des 4 clones, est longue de 2477 nucléotides. Elle présente une séquence nucléotidique identique à celle de l'ARN messager endothélial, en 3' de la position 228 du cDNA testiculaire. En 5' de cette position les deux ARN messagers testiculaires et endothéliaux divergent et la séquence spécifique testiculaire déterminée est de 228 nucléotides. Depuis le premier codon ATG jusqu'au codon stop, il existe une phase ouverte codant pour 732 acides aminés. L'analyse de la séquence peptidique déduite de la séquence nucléotidique montre qu'il existe après la méthionine de départ de la traduction, un peptide qui présente les caractéristiques d'un signal peptide et en utilisant le programme décrit par VON HEIJNE (Nucleic Acids Research, (1986), 14, 4683-4690) pour la prédiction des sites de coupure d'un signal peptide, le site de coupure du signal peptide se situerait entre l'acide aminé en position 21 et celui en position 22. A la suite de cette séquence signal, la séquence peptidique spécifique de l'ECA testiculaire est très riche en serine et en thréonine, sites potentiels de glycosylation, et ce type de séquence est évocateur de O-glycosylation groupée.

Les caractéristiques enzymatiques de l'enzyme testiculaire étant identiques à celle de l'enzyme endothéliale, notamment en ce qui concerne le Km, la Vmax, et Kcat sur l'angiotensine I, la comparaison de sa structure avec celle de l'enzyme endothéliale apporte des informations intéressantes concernant les relations structure activité dans la molécule d'ECA. En effet, l'enzyme endothéliale est constituée de deux grands domaines, très homologues entre eux. Chacun de ces domaines contient une courte séquence d'acides aminés consensus du site actif des métallopeptidases à zinc tels que la thermolysine, la collagénase ou l'endopeptidase neutre. L'enzyme de conversion testiculaire ne contient que le domaine carboxyterminal de l'ECA, endothéliale, ce qui semble indiquer que ce domaine à lui seul porte les structures responsables de l'activité enzymatique. Une autre hypothèse serait que l'enzyme testiculaire agit sous forme d'un dimère, cependant l'enzyme testiculaire native de lapin testée en condition non dénaturante par centrifugation en gradient de densité a bien un poids moléculaire inférieur à celui de l'ECA endothéliale.

## Revendications

1. Acide nucléique **caractérisé en ce qu'**il comprend une séquence codant pour l'enzyme de conversion de l'angiotensine testiculaire humaine consistant en la séquence nucléique de la figure 1, ou une partie de cette séquence, cette partie comprenant elle-même entre 15 et 201 nucléotides de la séquence nucléique délimitée par les nucléotides situés aux positions 29 et 229 de la figure 1, et **en ce qu'**il code pour un polypeptide, ce polypeptide étant:
- reconnu par des anticorps polyclonaux ou monoclonaux reconnaissant spécifiquement tout peptide issu de la séquence peptidique délimitée par les acides aminés situés aux positions 1 à 67 de la figure 1,
- et, le cas échéant, capable d'hydrolyser l'angiotensine I et/ou les kinines, notamment la bradykinine.

2. Acide nucléique **caractérisé en ce qu'**il hybride avec tout fragment de la séquence nucléotidique de la figure 1 qui est spécifique de l'enzyme de conversion de l'angiotensine testiculaire (ECA) humaine, ladite séquence spécifique étant délimitée par les nucléotides situés aux positions 29 et 229 de la figure 1, dans les conditions d'hybridation stringentes suivantes :
- dénaturation de l'acide nucléique (ADN) susceptible de s'hybrider avec celui de la figure 1, par traitement à l'aide d'une solution alcaline (0,5 MNaOH), suivie d'un passage à pH neutre,
- traitement de préhybridation du support (filtre de nitrocellulose ou membrane de nylon), sur lequel est fixé l'acide nucléique représenté sur la figure 1, à 50°C pendant 3 heures avec une solution ayant la composition suivante : 25 mM KPO₄, pH 7,4 ; 5 x SSC ; 5 x Denhardt's ; 50 µg/ml d'ADN de sperme de saumon soniqué ; 0,1% Sodium Dodecyl Sulfate (SDS ou NaDod SO₄⁻),
- remplacement de la solution de pré-hybridation au contact du support par une solution tampon ayant une composition identique à celle de pré-hybridation indiquée ci-dessus, (plus éventuellement 10% de Dextran), et comprenant l'acide nucléique de la figure 1 en tant que sonde marquée, notamment de manière radioactive, et préalablement dénaturée par un traitement à 90°C pendant 3 minutes,
- incubation pendant 12 heures à 60°C,
- lavages successifs avec les solutions suivantes :
. 2 x SSC, pendant 30 minutes à 60°C à 2 reprises,
. 1 x SSC, 0,1% SDS pendant 1 à 2 fois 15 minutes à 65°C.

3. Acide nucléique **caractérisé en ce qu'**il hybride avec tout fragment de la séquence nucléotidique de la figure 1 qui est spécifique de l'enzyme de conversion de l'angiotensine testiculaire (ECA) humaine, ladite séquence spécifique étant délimitée par les nucléotides situés aux positions 29 et 229 de la figure 1, dans des conditions non stringentes reprenant les caractéristiques essentielles des conditions stringentes définies dans la revendication 2, sauf pour ce qui concerne la température qui est de 40°C dans les conditions non stringentes, et les lavages successifs qui, dans les conditions non stringentes, sont réalisés à l'aide de 2 x SSC avec ou sans SDS à 45°C pendant 15 minutes à 2 reprises, et **en ce qu'**il code pour un polypeptide, ce polypeptide étant:
- reconnu par des anticorps polyclonaux ou monoclonaux reconnaissant spécifiquement la séquence peptidique délimitée par les acides aminés situés aux positions 1 à 67 de la figure 1,
- et, le cas échéant, capable d'hydrolyser l'angiotensine I et/ou les kinines, notamment la bradykinine..

4. Acide nucléique selon la revendication 1, **caractérisé en ce qu'**il comprend une séquence d'ADN délimitée par les nucléotides situés aux positions 92 et 2224 de la figure 1, codant pour l'ECA testiculaire humaine mature.

5. Acide nucléique selon la revendication 4, **caractérisé en ce que** ladite séquence d'ADN codant pour l'ECA testiculaire humaine mature est précédée par une séquence d'ADN délimitée par les nucléotides situés aux positions 29 et 91 de la figure 1, cette dernière codant pour un peptide signal de 21 acides aminés.

6. Acide nucléique selon la revendication 1, **caractérisé en ce qu'**il comprend une séquence d'ADN s'étendant entre, d'une part, le nucléotide situé entre les positions 1 à 92 et, d'autre part, le nucléotide situé entre les positions 229 à 2224 de la figure 1.

7. Acide nucléique selon la revendication 1, **caractérisé en ce qu'**il comprend une séquence d'ADN d'environ 15 à 201 nucléotides, issue de la séquence nucléique s'étendant entre le nucléotide situé à la position 29 et le nucléotide situé à la position 229 de la figure 1.

8. Acide nucléique selon la revendication 1, **caractérisé en ce qu'**il comprend une séquence d'ADN d'environ 15 à 135 nucléotides, issue de la séquence nucléique s'étendant entre le nucléotide situé à la position 92 et le nucléotide situé à la position 229 de la figure 1.

9. Polypeptide consistant en une séquence peptidique de 5 à 67 acides aminés, issue de la séquence peptidique délimitée par les acides aminés situés aux positions 1 et 67 de la figure 1.

10. Polypeptide consistant en une séquence peptidique de 5 à 45 acides aminés, issue de la séquence peptidique délimitée par les acides aminés situés aux positions 22 et 67 de la figure 1.

11. Polypeptide selon l'une des revendications 9 à 10 **caractérisé en ce qu'**il est couplé à la sérum-albumine bovine, à la KLH ou à la LPH.

12. Polypeptide comprenant une séquence peptidique de 5 à 67 acides aminés, issue de la séquence peptidique délimitée par les acides aminés situés aux positions 1 et 67 de la figure 1, **caractérisé en ce qu'**il est couplé à la sérum-albumine bovine, à la KLH ou à la LPH.

13. Polypeptide comprenant une séquence peptidique de 5 à 45 acides aminés, issue de la séquence peptidique délimitée par les acides aminés situés aux positions 22 et 67 de la figure 1, **caractérisé en ce qu'**il est couplé à la sérum-albumine bovine, à la KLH ou à la LPH.

14. Acide nucléique **caractérisé en ce qu'**il comprend une séquence nucléotidique selon l'une quelconque des revendications 1 à 8 associée avec un promoteur sous le contrôle duquel la transcription de ladite séquence est susceptible d'être effectuée et, le cas échéant, une séquence d'ADN codant pour des signaux de terminaison de la transcription.

15. Vecteur recombinant, notamment plasmide recombinant, ou virus recombinant, capable de transformer, ou d'infecter, une cellule hôte appropriée, notamment une cellule eucaryote, ledit vecteur contenant un acide nucléique selon l'une quelconque des revendications 1 à 8 sous le contrôle d'éléments de régulation permettant l'expression de ce fragment d'ADN dans la cellule hôte, notamment d'un promoteur reconnu par les polymérases de ladite cellule hôte.

16. Procédé de production de l'ECA testiculaire humaine, mature ou non, ou d'un polypeptide issu de l'ECA testiculaire humaine, ce procédé comprenant la transformation de cellules hôtes au moyen du vecteur selon la revendication 15, la mise en culture des cellules hôtes transformées dans un milieu de culture approprié et la récupération de l'enzyme à partir de ces cellules ou du milieu de culture.

17. Anticorps polyclonal ou monoclonal, **caractérisé en ce qu'**il est dirigé contre un polypeptide selon l'une quelconque des revendications 9 ou 10.

18. Sonde nucléotidique **caractérisée en ce qu'**elle est constituée par une séquence nucléotidique hybridant avec tout fragment de l'acide nucléique selon l'une quelconque des revendications 1 à 8 qui est spécifique de l'enzyme de conversion de l'angiotensine testiculaire (ECA) humaine, ladite séquence spécifique étant délimitée par les nucléotides situés aux positions 29 et 229 de la figure 1, dans les conditions définies dans la revendication 2.

19. Amorce nucléotidique **caractérisée en ce qu'**elle est constituée d'une succession d'environ 15 à 30 nucléotides issus d'une séquence nucléotidique selon l'une des revendications 1 à 8 qui est spécifique de l'enzyme de conversion de l'angiotensine testiculaire (ECA) humaine, ladite séquence spécifique étant délimitée par les nucléotides situés aux positions 29 et 229 de la figure 1, ou d'une séquence complémentaire de cette dernière.

20. Méthode de dépistage, ou de dosage, *in vitro* d'une ECA ou produit ayant *in vivo* les propriétés de l'ECA, et, plus particulièrement, de l'ECA testiculaire humaine, dans un prélèvement biologique susceptible de la contenir, **caractérisée en ce qu'**elle comprend :
- la mise en contact d'un anticorps selon la revendication 17, avec le susdit prélèvement biologique dans des conditions permettant la production éventuelle d'un complexe immunologique formé entre l'ECA ou produit qui en est dérivé et l'anticorps sus-mentionné;
- la détection du susdit complexe immunologique.

21. Méthode de dépistage, ou de dosage, *in vitro* d'une ECA ou produit ayant *in vivo* les propriétés de l'ECA, et, plus particulièrement, de l'ECA testiculaire humaine dans un prélèvement biologique susceptible de la contenir, **caractérisée en ce qu'**elle comprend:
- le cas échéant, l'amplification du nombre de copies de l'acide nucléique à détecter à l'aide d'un couple d'amorces selon la revendication 19,
- la mise en contact d'une sonde nucléotidique selon la revendication 18, avec le susdit prélèvement biologique dans des conditions permettant la production éventuelle d'un complexe d'hybridation formé entre la sonde et l'acide nucléique à détecter,
- la détection du susdit complexe d'hybridation.

22. Méthode de dépistage *in vitro* selon la revendication 20 ou 21, appliquée au diagnostic *in vitro* de la maturation des cellules germinales mâles humaines, des hypofertilités, et des stérilités masculines, ou encore des tumeurs testiculaires (ou séminomes).

23. Kit pour la mise en oeuvre d'une méthode de dépistage *in vitro* selon les revendications 20 et 22 comprenant :
- une quantité déterminée d'un anticorps selon la revendication 17 susceptible de donner lieu à une réaction immunologique avec un polypeptide à détecter selon l'une quelconque des revendications 9 à 13 ;
- avantageusement, un milieu approprié à la formation d'une réaction immunologique entre le polypeptide et l'anticorps susmentionnés ;
- avantageusement, des réactifs permettant la détection des complexes immunologiques formés entre le polypeptide et l'anticorps lors de la susdite réaction immunologique.

24. Kit pour la mise en oeuvre d'une méthode de dépistage *in vitro* selon la revendication 21 comprenant :
- le cas échéant, au moins un couple d'amorces selon la revendication 19,
- une quantité déterminée d'une sonde nucléotidique selon la revendication 18, susceptible de donner lieu à une réaction d'hybridation avec un acide nucléique selon l'une quelconque des revendications 1 à 8 ;
- avantageusement, un milieu approprié à la formation d'une réaction d'hybridation entre l'acide nucléique et la sonde susmentionnée ;
- avantageusement, des réactifs permettant la détection des complexes d'hybridation formés entre l'acide nucléique et la sonde lors de la susdite réaction d'hybridation.

25. Compositions pour le traitement de maladies inflammatoires ou infectieuses, notamment de la pancréatite aiguë, et des maladies où les kinines jouent un rôle pathogène, **caractérisées par** l'association d'un polypeptide obtenu par le procédé de la revendication 16, avec un véhicule pharmaceutiquement acceptable.

## Claims

1. Nucleic acid **characterized in that** it comprises a sequence encoding human testicular angiotensin converting enzyme consisting of the nucleic sequence of Figure 1, or part of this sequence, this part itself comprising between 15 and 201 nucleotides of the nucleic sequence delimited by the nucleotides situated at positions 29 and 229 of Figure 1, and **in that** it encodes a polypeptide, this polypeptide being:
- recognized by polyclonal or monoclonal antibodies specifically recognizing any peptide derived from the peptide sequence delimited by the amino acids situated at positions 1 to 67 of Figure 1,
- and, where appropriate, capable of hydrolysing angiotensin I and/or the kinins, in particular bradykinin.

2. Nucleic acid **characterized in that** it hybridizes with any fragment of the nucleotide sequence of Figure 1 which is specific for human testicular angiotensin converting enzyme (ACE), the said specific sequence being delimited by the nucleotides situated at positions 29 and 229 of Figure 1, under the following stringent hybridization conditions:
- denaturation of the nucleic acid (DNA) capable of hybridizing with that of Figure 1, by treatment with the aid of an alkaline solution (0.5 M NaOH), followed by passage to a neutral pH,
- prehybridization treatment of the support (nitrocellulose filter or nylon membrane) onto which the nucleic acid represented in Figure 1 is attached, at 50°C for 3 hours with a solution having the following composition: 25 mM KPO₄, pH 7.4; 5 × SSC; 5 × Denhardt's; 50 µg/ml of sonicated salmon sperm DNA; 0.1% sodium dodecyl sulphate (SDS or NaDod SO₄⁻),
- replacement of the prehybridization solution in contact with the support by a buffer solution having an identical composition to that of the prehybridization solution indicated above (plus optionally 10% Dextran) and comprising the nucleic acid of Figure 1 as a probe which is labelled, in particular radioactively, and denatured beforehand by a treatment at 90°C for 3 minutes,
- incubation for 12 hours at 60°C,
- successive washes with the following solutions:
• 2 × SSC, for 30 minutes at 60°C twice,
• 1 × SSC, 0.1% SDS for 1 to 2 times 15 minutes at 65°C.

3. Nucleic acid **characterized in that** it hybridizes with any fragment of the nucleotide sequence of Figure 1 which is specific for human testicular angiotensin converting enzyme (ACE), the said specific sequence being delimited by the nucleotides situated at positions 29 and 229 of Figure 1, under nonstringent conditions which have the essential characteristics of the stringent conditions defined in Claim 2, except as regards the temperature which is 40°C under nonstringent conditions, and the successive washes which, under nonstringent conditions, are carried out with the aid of 2 × SSC with or without SDS at 45°C for 15 minutes twice, and **in that** it encodes a polypeptide, this polypeptide being:
- recognized by polyclonal or monoclonal antibodies specifically recognizing the peptide sequence delimited by the amino acids situated at positions 1 to 67 of Figure 1,
- and, where appropriate, capable of hydrolysing angiotensin I and/or the kinins, in particular bradykinin.

4. Nucleic acid according to Claim 1, **characterized in that** it comprises a DNA sequence delimited by the nucleotides situated at positions 92 and 2224 of Figure 1, encoding mature human testicular ACE.

5. Nucleic acid according to Claim 4, **characterized in that** said DNA sequence encoding mature human testicular ACE is preceded by a DNA sequence delimited by the nucleotides situated at positions 29 and 91 of Figure 1, the latter encoding a signal peptide of 21 amino acids.

6. Nucleic acid according to Claim 1, **characterized in that** it comprises a DNA sequence extending between, on the one hand, the nucleotide situated between positions 1 and 92 and, on the other hand, the nucleotide situated between positions 229 and 2224 of Figure 1.

7. Nucleic acid according to Claim 1, **characterized in that** it comprises a DNA sequence of about 15 to 201 nucleotides, derived from the nucleic sequence extending between the nucleotide situated at position 29 and the nucleotide situated at position 229 of Figure 1.

8. Nucleic acid according to Claim 1, **characterized in that** it comprises a DNA sequence of about 15 to 135 nucleotides, derived from the nucleic sequence extending between the nucleotide situated at position 92 and the nucleotide situated at position 229 of Figure 1.

9. Polypeptide consisting of a peptide sequence of 5 to 67 amino acids, derived from the peptide sequence delimited by the amino acids situated at positions 1 and 67 of Figure 1.

10. Polypeptide consisting of a peptide sequence of 5 to 45 amino acids, derived from the peptide sequence delimited by the amino acids situated at positions 22 and 67 of Figure 1.

11. Polypeptide according to one of Claims 9 to 10, **characterized in that** it is coupled to bovine serum albumin, to KLH or to LPH.

12. Polypeptide comprising a peptide sequence of 5 to 67 amino acids, derived from the peptide sequence delimited by the amino acids situated at positions 1 and 67 of Figure 1, **characterized in that** it is coupled to bovine serum albumin, to KLH or to LPH.

13. Polypeptide comprising a peptide sequence of 5 to 45 amino acids, derived from the peptide sequence delimited by the amino acids situated at positions 22 and 67 of Figure 1, **characterized in that** it is coupled to bovine serum albumin, to KLH or to LPH.

14. Nucleic acid **characterized in that** it comprises a nucleotide sequence according to any one of Claims 1 to 8, combined with a promoter under the control of which the transcription of the said sequence is capable of being carried out and, where appropriate, a DNA sequence encoding the signals for termination of transcription.

15. Recombinant vector, in particular a recombinant plasmid, or a recombinant virus, capable of transforming, or of infecting, an appropriate host cell, in particular a eukaryotic cell, the said vector containing a nucleic acid according to any one of Claims 1 to 8 under the control of regulatory elements allowing the expression of this DNA fragment in the host cell, in particular of a promoter recognized by the polymerases of the said host cell.

16. Method for producing human testicular ACE, mature or otherwise, or a polypeptide derived from human testicular ACE, this method comprising the transformation of host cells by means of the vector according to Claim 15, culturing the transformed host cells in an appropriate culture medium and recovering the enzyme from these cells or from the culture medium.

17. Polyclonal or monoclonal antibody, **characterized in that** it is directed against a polypeptide according to either of Claims 9 and 10.

18. Nucleotide probe **characterized in that** it consists of a nucleotide sequence which hybridizes with any fragment of the nucleic acid according to any one of Claims 1 to 8 which is specific for human testicular angiotensin converting enzyme (ACE), the said specific sequence being delimited by the nucleotides situated at positions 29 and 229 of Figure 1, under the conditions defined in Claim 2.

19. Nucleotide primer **characterized in that** it consists of a succession of about 15 to 30 nucleotides derived from a nucleotide sequence according to one of Claims 1 to 8 which is specific for human testicular angiotensin converting enzyme (ACE), the said specific sequence being delimited by the nucleotides situated at positions 29 and 229 of Figure 1, or from a sequence complementary thereto.

20. Method for screening, or for assaying, *in vitro* an ACE or a product having *in vivo* the properties of ACE, and more particularly of human testicular ACE, in a biological sample capable of containing it, **characterized in that** it comprises:
- bringing an antibody according to Claim 17 into contact with the abovementioned biological sample under conditions allowing the possible production of an immunological complex formed between the ACE or a product which is derived therefrom and the abovementioned antibody;
- detecting the abovementioned immunological complex.

21. Method for screening, or for assaying, in vitro an ACE or a product having in vivo the properties of ACE, and more particularly of human testicular ACE, in a biological sample capable of containing it, **characterized in that** it comprises:
- where appropriate, amplifying the number of copies of nucleic acid to be detected with the aid of a pair of primers according to Claim 19,
- bringing a nucleotide probe according to Claim 18 into contact with the abovementioned biological sample under conditions allowing the possible production of a hybridization complex formed between the probe and the nucleic acid to be detected,
- detecting the abovementioned hybridization complex.

22. Method for screening *in vitro* according to Claim 20 or 21, applied to the diagnosis *in vivo* of the maturation of human male germ cells, of hypofertilities, and of male sterilities, or alternatively of testicular tumours (or seminomas).

23. Kit for carrying out a method for screening *in vitro* according to Claims 20 and 22 comprising:
- a determined quantity of an antibody according to Claim 17 capable of giving rise to an immunological reaction with a polypeptide to be detected according to any one of Claims 9 to 13;
- advantageously, a medium appropriate for the formation of an immunological reaction between the abovementioned polypeptide and antibody;
- advantageously, reagents allowing the detection of the immunological complexes formed between the polypeptide and the antibody during the abovementioned immunological reaction.

24. Kit for carrying out a method for screening *in vitro* according to Claim 21 comprising:
- where appropriate, at least one pair of primers according to Claim 19,
- a determined quantity of a nucleotide probe according to Claim 18, capable of giving rise to a hybridization reaction with a nucleic acid according to any one of Claims 1 to 8;
- advantageously, a medium appropriate for the formation of a hybridization reaction between the nucleic acid and the abovementioned probe;
- advantageously, reagents allowing the detection of the hybridization complexes formed between the nucleic acid and the probe during the abovementioned hybridization reaction.

25. Compositions for the treatment of inflammatory or infectious diseases, in particular of acute pancreatitis, and of diseases where kinins play a pathogenic role, **characterized by** the combination of a polypeptide obtained by the method of Claim 16, with a pharmaceutically acceptable vehicle.

## Patentansprüche

1. Nucleinsäure, **dadurch gekennzeichnet, dass** sie eine Sequenz umfasst, die das menschliche Testikei-Angiotensin-Converting-Enzym codiert, bestehend aus der Nucleinsäuresequenz wie in Figur 1 dargestellt oder aus einem Teil dieser Sequenz, wobei dieser Teil selbst zwischen 15 und 201 Nucleotide der Nucleinsäuresequenz umfasst, die begrenzt wird durch die Nucleotide, die sich in den Positionen 29 und 229 der Figur 1 befinden, und dass sie ein Polypeptid codiert, wobei das Polypeptid:
- von polyclonalen oder monoclonalen Antikörpern erkannt wird, die insbesondere alle von der Peptidsequenz stammenden Peptide erkennen, die von den Aminosäuren begrenzt wird, die sich in den Positionen 1 bis 67 der Figur 1 befinden,
- und, gegebenenfalls, fähig ist, das Angiotensin I und/oder die Kinine, insbesondere Bradykinin, zu hydrolysieren.

2. Nucleinsäure, **dadurch gekennzeichnet, dass** sie mit jedem Fragment der Nucleotidsequenz der Figur 1 hybridisiert, die spezifisch ist für das menschliche Testikei-Angiotensin-Converting-Enzym (ACE), wobei die spezifische Sequenz begrenzt ist durch die Nucleotide, die sich in den Positionen 29 und 229 der Figur 1 befinden, unter den folgenden stringenten Hybridisierungsbedingungen:
- Denaturierung der Nucleinsäure (DNA), die geeignet ist zur Hybridisierung mit der Nucleinsäure der Figur 1, durch Behandlung mit Hilfe einer Alkalilösung (0,5 M NaOH), gefolgt von einer Passage bei einem neutralem pH-Wert,
- Prähybridisierungsbehandlung des Trägers (Nitrocellulosefilter oder Nylonmembran), auf dem die Nucleinsäure nach Figur 1 fixiert ist, bei 50°C über 3 Stunden mit einer Lösung, die die folgende Zusammensetzung hat: 25 mM KPO₄, pH 7,4; 5 x SSC; 5 x Denhardts; 50 µg/ml DNA von beschalltem Lachssperma; 0,1% Natriumdodecylsulfat (SDS oder NaDod SO₄⁻),
- Ersetzen der Prähybridisierungslösung mit Trägerkontakt durch eine Pufferlösung, die eine identische Zusammensetzung hat wie die vorstehend beschriebene Prähybridisierungslösung, (und gegebenenfalls 10% Dextran) und die die Nucleinsäure der Figur 1 als, insbesondere radioaktiv, markierte Sonde umfasst, und die vorher durch eine Behandlung bei 90°C über 3 Minuten denaturiert wurde,
- Inkubation über 12 Stunden bei 60°C,
- wiederholtes Waschen mit den folgenden Lösungen:
. 2 x SSC, über 30 Minuten bei 60°C mit 2 Wiederholungen,
. 1 x SSC, 0,1% SDS 1 bis 2 Mal 15 Minuten bei 65°C.

3. Nucleinsäure, **dadurch gekennzeichnet, dass** sie mit jedem Fragment der Nucleotidsequenz der Figur 1 hybridisiert, die spezifisch für das menschliche Testikei-Angiotensin-Converting-Enzym (ACE) ist, wobei die spezifische Sequenz begrenzt wird durch die Nucleotide, die sich in den Positionen 29 und 229 der Figur 1 befinden, unter nicht stringenten Bedingungen, wobei die essentiellen Merkmale der in Anspruch 2 definierten stringenten Bedingungen übernommen werden, außer im Bezug auf die Temperatur, die unter den nicht stringenten Bedingungen 40°C beträgt, und auf das wiederholte Waschen, das unter nicht stringenten Bedingungen mit Hilfe von 2 x SSC durchgeführt wird, mit oder ohne SDS bei 45°C über 15 Minuten mit 2 Wiederholungen, und **dadurch gekennzeichnet, dass** sie ein Polypeptid codiert, wobei das Polypeptid:
- von polyclonalen oder monoclonalen Antikörpern erkannt wird, die insbesondere die Peptidsequenz erkennen, die von den Aminosäuren begrenzt wird, die sich in den Positionen 1 bis 67 der Figur 1 befinden,
- und, gegebenenfalls, fähig ist, das Angiotensin I und/oder die Kinine, insbesondere Bradykinin, zu hydrolysieren.

4. Nucleinsäure nach Anspruch 1, **dadurch gekennzeichnet, dass** sie eine DNA-Sequenz umfasst, die von den Nucleotiden begrenzt wird, die sich in den Positionen 92 und 2224 der Figur 1 befinden, codierend das reife ACE des menschlichen Testikels.

5. Nucleinsäure nach Anspruch 4, **dadurch gekennzeichnet, dass** der DNA-Sequenz, die das reife ACE des menschlichen Testikels codiert, eine DNA-Sequenz vorausgeht, begrenzt durch die Nucleotide, die sich in den Positionen 29 und 91 der Figur 1 befinden, die wiederum ein Signalpeptid mit 21 Aminosäuren codiert.

6. Nucleinsäure nach Anspruch 1, **dadurch gekennzeichnet, dass** sie eine DNA-Sequenz umfasst, die sich erstreckt zwischen auf der einen Seite dem Nucleotid, das sich zwischen den Positionen 1 bis 92 befindet, und auf der anderen Seite dem Nucleotid, das sich zwischen den Positionen 229 bis 2224 der Figur 1 befindet.

7. Nucleinsäure nach Anspruch 1, **dadurch gekennzeichnet, dass** sie eine DNA-Sequenz von etwa 15 bis 201 Nucleotiden umfasst, die hervorgegangen ist aus der Nucleotidsequenz, die sich erstreckt zwischen dem Nucleotid, das sich in der Position 29 befindet, und dem Nucleotid, das sich in der Position 229 der Figur 1 befindet.

8. Nucleinsäure nach Anspruch 1, **dadurch gekennzeichnet, dass** sie eine DNA-Sequenz von etwa 15 bis 135 Nucleotiden umfasst, die hervorgegangen ist aus der Nucleotidsequenz, die sich erstreckt zwischen dem Nucleotid, das sich in der Position 92 befindet, und dem Nucleotid, das sich in der Position 229 der Figur 1 befindet.

9. Polypeptid, bestehend aus einer Peptidsequenz von 5 bis 67 Aminosäuren, die hervorgegangen ist aus der Peptidsequenz, die begrenzt wird von den Aminosäuren, die sich in den Positionen 1 und 67 der Figur 1 befinden.

10. Polypeptid, bestehend aus einer Peptidsequenz von 5 bis 45 Aminosäuren, die hervorgegangen ist aus der Peptidsequenz, die begrenzt wird von den Aminosäuren, die sich in den Positionen 22 und 67 der Figur 1 befinden.

11. Polypeptid nach einem der Ansprüche 9 bis 10, **dadurch gekennzeichnet, dass** es mit Rinderserumalbumin, KLH oder LPH gekoppelt ist.

12. Polypeptid, umfassend eine Peptidsequenz aus 5 bis 67 Aminosäuren, die hervorgegangen ist aus der Peptidsequenz, die begrenzt wird durch die Aminosäuren, die sich in den Positionen 1 und 67 der Figur 1 befinden, **dadurch gekennzeichnet, dass** es an Rinderserumalbumin, KLH oder LPH gekoppelt ist.

13. Polypeptid, umfassend eine Peptidsequenz aus 5 bis 45 Aminosäuren, die hervorgegangen ist aus der Peptidsequenz, die begrenzt wird durch die Aminosäuren, die sich in den Positionen 22 und 67 der Figur 1 befinden, **dadurch gekennzeichnet, dass** es an Rinderserumalbumin, KLH oder LPH gekoppelt ist.

14. Nucleinsäure, **dadurch gekennzeichnet, dass** sie eine Nucleotidsequenz nach einem der Ansprüche 1 bis 8 umfasst, die mit einem Promotor verbunden ist, unter dessen Kontrolle die Transkription der Sequenz durchgeführt werden kann, und gegebenenfalls eine DNA-Sequenz, die Transkriptionsterminationssignale codiert.

15. Rekombinanter Vektor, insbesondere ein rekombinantes Plasmid oder ein rekombinantes Virus, das in der Lage ist, eine geeignete Wirtszelle zu transformieren oder zu infizieren, insbesondere eine eukaryotische Zelle, wobei der Vektor eine Nucleinsäure nach einem der Ansprüche 1 bis 8 enthält, unter Kontrolle von Regulationselementen, die die Expression dieses DNA-Fragments in der Wirtszelle erlauben, insbesondere eines Promotors, der von den Polymerasen der Wirtszelle erkannt wird.

16. Verfahren zur Herstellung von reifem oder nicht reifem menschlichem Testikel-ACE, oder eines Polypeptids, das aus dem menschlichen Testikel-ACE hervorgegangen ist, wobei dieses Verfahren die Transformation von Wirtszellen mit Hilfe des Vektors nach Anspruch 15, die Züchtung von transformierten Wirtszellen in einem geeigneten Kulturmedium und die Gewinnung des Enzyms aus diesen Zellen oder aus diesem Kulturmedium umfasst.

17. Polyclonaler oder monoclonaler Antikörper, **dadurch gekennzeichnet, dass** er gegen ein Polypeptid nach einem der Ansprüche 9 oder 10 gerichtet ist.

18. Nucleotidsonde, **dadurch gekennzeichnet, dass** sie zusammengesetzt ist aus einer Nucleotidsequenz, die mit jedem Fragment der Nucleinsäure nach einem der Ansprüche 1 bis 8 hybridisiert, die spezifisch ist für das menschliche Testikel-ACE, wobei die spezifische Sequenz begrenzt ist durch die Nucleotide, die sich in den Positionen 29 und 229 der Figur 1 befinden, unter den Bedingungen wie in Anspruch 2 definiert.

19. Nucleotidprimer, **dadurch gekennzeichnet, dass** er zusammengesetzt ist aus einer Folge von etwa 15 bis 30 Nucleotiden, die aus einer Nucleotidsequenz nach einem der Ansprüche 1 bis 8 hervorgegangen sind, die für das menschliche Testikel-ACE spezifisch ist, wobei die spezifische Sequenz begrenzt ist von den Nucleotiden, die sich in den Positionen 29 und 229 der Figur 1 befinden, oder für eine komplementäre Sequenz der Letzteren.

20. Verfahren zur *in-vitro*-Erkennung oder Dosierung eines ACE oder eines Produktes, das *in vivo* die Eigenschaften des ACE und, insbesondere, des menschlichen Testikel-ACE hat, in einer biologischen Probe, die dieses enthalten kann, **dadurch gekennzeichnet, dass** es umfasst:
- Inkontaktbringen eines Antikörpers nach Anspruch 17 mit der obengenannten biologischen Probe unter Bedingungen, die die mögliche Produktion eines immunologischen Komplexes zwischen dem ACE oder dem davon abgeleiteten Produkt und dem obengenannten Antikörper erlauben;
- Nachweis des obengenannten immunologischen Komplexes.

21. Verfahren zur *in-vitro-*Erkennung oder Dosierung eines ACE oder eines Produktes, das *in vivo* die Eigenschaften des ACE und, insbesondere, des menschlichen Testikel-ACE hat, in einer biologischen Probe, die dieses enthalten kann, **dadurch gekennzeichnet, dass** es umfasst:
- gegebenenfalls Amplifikation der Anzahl der Kopien der nachzuweisenden Nucleinsäure mit Hilfe eines Primerpaares nach Anspruch 19,
- Inkontaktbringen einer Nucleotidsonde nach Anspruch 18 mit der obengenannten biologischen Probe unter Bedingungen, die die mögliche Produktion eines Hybridisierungskomplexes zwischen der Sonde und der nachzuweisenden Nucleinsäure erlauben,
- Nachweis des obengenannten Hybridisierungskomplexes.

22. Verfahren zur *in*-*vitro*-Erkennung nach Anspruch 20 oder 21, angewendet bei der *in-vitro*-Diagnostik im Zusammenhang mit der Reifung von männlichen Keimzellen beim Menschen, mit Fällen von Hypofertilität und Sterilität beim Mann oder auch im Bezug auf Testikeltumoren (oder Seminomen).

23. Kit zur Anwendung eines Verfahrens zum *in-vitro*-Erkennen nach den Ansprüchen 20 und 22, umfassend:
- eine bestimmte Menge eines Antikörpers nach Anspruch 17, der eine Immunreaktion mit einem nachzuweisenden Polypeptid nach einem der Ansprüche 9 bis 13 hervorrufen kann;
- vorzugsweise ein geeignetes Medium für die Entstehung einer Immunreaktion zwischen dem Polypeptid und den obengenannten Antikörpern;
- vorzugsweise Reagenzien, die den Nachweis der Immunkomplexe erlauben, die während der obengenannten Immunreaktion zwischen dem Polypeptid und dem Antikörper gebildet wurden.

24. Kit zur Anwendung eines Verfahrens zum *in-vitro*-Erkennen nach Anspruch 21, umfassend:
- gegebenenfalls mindestens ein Primerpaar nach Anspruch 19,
- eine bestimmte Menge einer Nucleotidsonde nach Anspruch 18, die eine Hybridisierungsreaktion mit einer Nucleinsäure nach einem der Ansprüche 1 bis 8 hervorrufen kann;
- vorzugsweise ein zur Bildung einer Hybridisierungsreaktion zwischen der Nucleinsäure und der obengenannten Sonde geeignetes Medium;
- vorzugsweise Reagenzien, die den Nachweis der Hybridisierungskomplexe erlauben, die während der obengenannten Hybridisierungsreaktion zwischen der Nucleinsäure und der Sonde gebildet wurden.

25. Zusammensetzungen zur Behandlung von entzündlichen oder infektiösen Erkrankungen, besonders von akuter Bauchspeicheldrüsenentzündung und von Erkrankungen, bei denen Kinine eine pathogene Rolle spielen, **gekennzeichnet durch** das Zusammengeben eines nach dem Verfahren nach Anspruch 16 erhaltenen Polypeptids mit einem pharmazeutisch verträglichen Träger.
